Europäisches Patentamt

European Patent Office

Office européen des brevets

(11) Publication number: **0 037 273**
**B1**

## EUROPEAN PATENT SPECIFICATION

(45) Date of publication of patent specification: **14.01.87**

(21) Application number: **81301360.4**

(22) Date of filing: **27.03.81**

(51) Int. Cl.⁴: **C 12 N 15/00,** C 12 N 1/20,
C 12 P 21/02

(54) **Modification of micro-organisms.**

(30) Priority: **28.03.80 GB 8010413**

(43) Date of publication of application:
**07.10.81 Bulletin 81/40**

(45) Publication of the grant of the patent:
**14.01.87 Bulletin 87/03**

(84) Designated Contracting States:
**AT BE CH DE FR GB IT LI LU NL SE**

(56) References cited:
**EP-A-0 035 831**
**GB-A-2 003 926**

**CHEMICAL ABSTRACTS, vol. 91, no. 21,
November 19, 1979, page 341, abstract
171422x Columbus, Ohio, US K. JEYASEELAN
et al.: "Transfer of antibiotic resistance to
facultative methylotrophs with plasmid R68.
45"**

(73) Proprietor: **IMPERIAL CHEMICAL INDUSTRIES
PLC**
**Imperial Chemical House Millbank**
**London SW1P 3JF (GB)**

(72) Inventor: **Barth, Peter Thomas**
**Alvanley House Alvanley Road**
**Helsby Via Warrington WA6 9PX (GB)**
Inventor: **Atherton, Keith Thurston**
**9 Tarvin CLose**
**Runcorn Cheshire (GB)**
Inventor: **Hennam, John Frederick**
**38 Solway Grove Beechwood West**
**Runcorn Cheshire (GB)**
Inventor: **Sharpe, Geoffrey Sidney**
**4 Elmcroft Road**
**North Kilworth Leicestershire LE17 6BX (GB)**

(74) Representative: **Bate, Bernard James et al**
**Imperial Chemical Industries PLC**
**Legal Department: Patents PO Box 6**
**Welwyn Garden City Herts, AL7 1HD (GB)**

Courier Press, Leamington Spa, England.

**0 037 273**

(56) References cited:

13th INTERNATIONAL TNO CONFERENCE
"Biotechnology a Hidden Past, a Shining
Future" March 27 and 28, 1980 Published by the
Netherlands Central Organisation for Applied
Scientific Research TNO, The Hague, NL P.J.
SENIOR et al.: "The ICI Single Cell Protein
Process", pages 97-101

NATURE, vol. 287, no. 5781, October 2, 1980
CHESHAM, BUCKS, GB J.D. WINDASS et al.:
"Improved conversion of methanol to single
cell protein by Methylophilus methylotrophus",
pages 396-401

**Description**

This invention relates to genetically modified microorganisms, to a method for their production to the use of such organisms, and to novel plasmids.

Most genetic manipulation of microorganisms (i.e. modification of their genetic material, usually by insertion of foreign DNA) has involved the E.coli K12 organism or its biologically attenuated variants. E.coli, however, is found in man and there are advantages in employing an organism that is not normally found in man and which is less likely to infect him.

Genetic manipulation of microorganisms is commonly employed with the object of introducing into a cell 'foreign' genetic material (DNA) the expression of which enables that cell to produce metabolites, for example biologically active molecules, whether antibiotic, hormonal, antiviral, or having other physiological or biological activity, which the cell in its 'natural', i.e. unmodified state, is unable to produce. To this end we have found it advantageous to employ as the host organism (that is, the organism into which the foreign DNA is ultimately inserted and which expresses it in the form of the desired metabolite) a bacterium which is capable of using as its sole or principal carbon source either inorganic carbon, for example in the form of carbon dioxide or a bicarbonate (i.e. an autotrophic bacterium), or methane or 'methyl' carbon (i.e. a methylotrophic bacterium).

A particularly valuable attribute of these organisms is their ability, when modified by the insertion of suitable foreign eukaryotic DNA, of expressing metabolites similar to or identical with that derived from higher, i.e. non-bacterial organisms, while utilising relatively cheap sources of carbon.

The foreign DNA may be naturally occurring, in the sense that it occurs, at least in its major sequence, in a different, preferably a higher organism, or it may be 'synthetic', that is synthesised to match or copy a naturally occurring DNA at least in respect of a major part of its functional sequence.

Accordingly, the invention provides an autotrophic or a methylotrophic bacterium which (a) comprises foreign genetic material which (i) in its unmodified state the bacterium would not comprise and (ii) has been inserted into a naturally occurring or composite plasmid and (b) is capable of effecting expression of the said foreign genetic material with generation of a polyamide which the foreign genetic material encodes and which it is unmodified state the bacterium would not generate with the proviso that the foreign genetic material is not a microbial g*d*h gene. Preferably the foreign genetic material is eukaryotic DNA.

Especially useful in a commercial sense are bacterial metabolites having an effect in mammals, and particularly in man, and these may conveniently be obtained by expression by bacteria comprising genetic material derived from a mammalian, preferably human, source.

Thus, a preferred microorganism according to the invention, is an autotrophic or a methylotrophic bacterium comprising eukaryotic, preferably mammalian, and partiuclarly preferably, human DNA.

Methods for the introduction of foreign genetic material into bacteria have been widely described in the literature, but we have found that the infection of the microorganisms of the invention is most conveniently carried out using a plasmid of one of incompatibility groups N, P, Q or W carrying the genetic material which it is desired to introduce the bacterial cell.

Accordingly, there is provided a method for the introduction of foreign genetic material into a cell of a species of an autotrophic or methylotrophic bacterium characterised in that the cell is infected with a plasmid of, or derived from, one of the incompatibility groups N, P, Q or W into which the foreign genetic material has been inserted, with the proviso that the foreign genetic material is not a microbial g*d*h gene. Plasmids of group Q are preferred.

The concept of incompatability groups is discussed in "DNA Insertion Elements, Plasmids and Episomes" Ed. by Bukhari, Shapiro and Adhya, Publ. Cold Spring Harbour Laboratory 1977 at p601 *et seq* (see also tables of plasmids in incompatibility groups at pp 607 to 668).

While many broad host-range conjugative plasmids such as RP4 (of the Inc P group 1) can be used for cloning, they are relatively large, exist in fairly low copy number and may, therefore, not be ideal for some cloning purposes. Preferably small, high copy number, broad host-range plasmids are used for cloning. R300B (of the Inc Q group) is an example of this category, but despite its advantages as a cloning vector it has relatively few sites for the restriction enzymes used for cloning.

It is therefore, sometimes advantageous to construct a composite plasmid capable of both stable maintenance in the host and containing a convenient and suitable expression site for foreign (especially eukaryotic) DNA in the organism. This may conveniently be accomplished by introducing segments of DNA from other plasmids containing both a selectable marker (e.g. Antibiotic Resistance) and suitable restriction enzyme sites. The method of constructing such a composite plasmid is preferably one of broad applicability, e.g. capable of making composite plasmids based on a wide range of plasmids mentioned above and on a wide range of expressing sites (e.g. within an antibiotic resistance gene; linked to a strong promoter for the bacterium or within a transposon). This aspect is exemplifed in the construction of the pGSS derivative pGSS15 described below.

In the performance of this aspect of the invention, a naturally occurring composite plasmid of one of the incompatibility groups, N, P, Q or W is modified by incorporation of genetic materials derived from a "donor", i.e. material which it is desired to introduce into the host bacterium. This may be effected conveniently by a process comprising the steps of cleaving the plasmid DNA, bringing the fragments so

# 0 037 273

obtained into admixture with fragments of host DNA obtained by cleaving host DNA material with similar DNA cleaving means and effecting random annealing of the mixture of DNA fragments so that a plasmid is obtained containing both plasmid and host DNA. This general procedure is well known and is further described and discussed for example in our UK Patent No. 1521032. Insertion of the donor genetic materials may be into a naturally occurring plasmid or into a plasmid which itself is already a composite of DNA from different sources.

The donor may be any suitable source, comprising DNA coding for the desired metabolite. The treatment required to prepare the donor material for transfer, for example extraction of DNA from the cells, may differ in different species, but such techniques are now well characterised and will be familar to the skilled man.

Several examples of the methylotrophic class of bacteria are known and have been described in the literature, for example *Methylophilus* spp, *Psuedomonas* spp, *Alcanigenes* spp, and *Methylomonas* spp. Specifically we may mention as examples

| | |
|---|---|
| *Methylotrophus methylophilus.* (AS1) | — NCIB 10508—10515 and 10592—10596, all inclusive;<br>— NRRL B5352—B5364 inclusive;<br>— FRI 1215—1227 inclusive; |
| *Pseudomonas methylonica* | — the characteristics of which are described in UK Specification No 1451020 and a culture of which is deposited as follows:<br>FRI 2247 and 2248 |
| *Pseudomonas methanolica* | — UK Specification No 1352728<br>— ATCC 21704; |
| *Pseudomonas sp* | — UK Specification No 1326582<br>— ATCC 21438 and 21439; |
| *Methylomonas methanolica* | — UK Specification No 1420264<br>— NRRL B5458; |
| *Pseudomonas utilis* | — UK Specification No 1444072<br>— FRI 1690 and 1691; |
| *Pseudomonas inaudita* | — UK Specification No 1444072<br>— FRI 1693 and 1694; |
| *Methylomonas clara* | — USP 4166004 — ATCC 31226. |

NB: NC1B is the National Collection of Industrial Bacteria, Aberdeen; NRRL is the Collection maintained by the US Department of Agriculture, Peoria, Illinois; ATCC is the American Type Culture collection; FRI is the Fermentation Research Institute, Japan.

Typically, such microorganisms may be aerobically cultured in an aqueuous medium containing methanol as a source of assimilable carbon, together with necessary inorganic nutrients.

Of the methylotrophes, our preferred microorganism for the purpose of the invention is *Methylophilus* and specifically *M.methylotrophus.* This organism, we have found, can be grown rapidly and efficiently, its toxicology has been investigated at length, and because it does not infect man and the higher organisms it is particularly safe for large scale biosynthesis, and its genetic composition is well known.

Many autotrophic bacteria are known and have been described in the literature. Examples are *Thiobacillus* spp, *Hydrogenomonas* spp, *Chromatrium* spp, *Anacystis* spp, *Rhodospirillum* spp, *Nitrobacter* spp, *Rhodopseudomonas* spp, and *Paracoccus* spp.

Both anaerobic and aerobic organisms are known, as well as both facultative and obligate organisms; in general we prefer to use obligate organisms as these allow greater control over processes involving them.

Selection of an appropriate organism, for a particular process, whether autotrophic or methylotrophic will obviously be made in the light of the characterisation of the organism and the requirements of the process contemplated. Selection of organisms will present no difficulty to the skilled man in the light of the information available in the literature.

4

Construction and use of a Composite Plasmid-pGSS15

Partially HaeII digested pBR322 DNA was mixed with partially HaeII digested R300B DNA, and treated with T4 DNA ligase at 10°C overnight (16 hours). This ligated mixture was then introduced into *E.coli* strain WJB654 by transformation of calcium treated cells and plated on L-agar containing tetracycline. Resistant colonies appeared after overnight incubation of the plates at 37°C and were replica plated onto medium contianing ampicillin, streptomycin or sulphonamides. Colonies that had acquired resistance to all four antibiotics were picked off and grown separately, with selection for at least one of the antibiotics.

Small scale cleared lysates were prepared of several colonies and the plasmid DNA analysed by gel electrophoresis. Colonies that contained plasmid DNA larger than the parent R300B DNA were picked and grown in liquid culture (L-broth) overnight and plasmid DNA isolated and purified by banding in cesium chloride equilibrium gradients. Restriction analysis of one of the plasmids, designated pGSS6, revealed the structure shown in Figure 1 i.e. a co-integrate of pBR322 and R300B.

pGSS6 was treated with restriction endonuclease *Eco*RI and the cut DNA was treated with T4 DNA ligase at low DNA concentration (1—5 µg/ml) to favour self-annealing of the fragments. The ligated DNA was transformed into *E.coli* WJB654 and some of the cells plated on medium containing ampicillin and some on medium containing tetracycline. After replica plating both sets of resistant progeny to look for other antibiotic resistances, two classes were isolated that were different from the parent pGSS6.

Once class was resistant to ampicillin and sulphonamides but sensitive to tetracycline and streptomycin, and the other class was resistant to tetracycline and streptomycin but sensitive to ampicillin and sulphonamides. The plasmids isolated from these two classes were designated pGSS9 and pGSS8 respectively (Figure 2). pGSS8 contains the tetracycline resistance gene from pBR322 on the wide host range replicon of R300B.

When PBR322 was digested to completion with the restriction endonuclease HaeII, a 1.87kb fragment was produced which carried the gene for β-lactamase. This fragment was inserted to pGSS8 by mixing totally HaeII digested pBR322 with partially HaeII digested pGSS8, treating with T4 DNA ligase then transforming *E.coli* strian NM36 and plating on L-agar containing ampicillin. Resistant colonies were checked for additional resistance to tetracycline and streptomycin. One colony was resistant to ampicillin and tetracycline, but sensitive to streptomycin. Plasmid DNA was isolated and analysed by restriction mapping, showing that the junction between the β-lactamase gene and the tetracycline had been reconstructed exactly at it appeared in pBR322. For this to happen, required a deletion of at least one HaeII fragment from pGSS8 to allows the *tet* gene to be reconstituted by the small piece of this gene carried by the β-lactamase HaeII fragment. This plasmid is designated pGSS15 (Figure 3).

Both pGSS8 and pGSS15 can be mobilized into AS1, and other organisms by conjugal transfer mediated by a conjugative plasmid. To introduce the "helper" conjugative plasmid into the *E.coli* strain carrying pGSS8 or pGSS15, cells of the latter strains would be spread over half of selective agar plates (i.e. the medium would not allow donor or recipient cells to grow, but would allow recipient containing newly acquied plasmid to grow) and donor cells streaked in e.g. WJB654 (pGSS15) cells (50 µl of ∿ $10^8$ cells/ml) were spread over half a glucose minimal agar plate containing 20 µg/ml streptomycin and 50 µg/ml methionine (Streptomycin prevents WJB654 (pGSS15) from growing). Fresh cells of BW162 (R64 drd 11) were streaked across from the clean side of the plate into the WJB654 (pGSS15) cells using a wire loop. (BW162 will not grow on glucose minimal medium with methionine, it requried threonine and leucine). After incubation at 37°C for 36—48 hours streptomycin resistant colonies of WJB654 (pGSS15/R64 drd 11) appeared and could be picked off and purified to single colonies. In a similar way, WJB654 (pGSS15/R64 drd 11) was streaked across into AS1 cells spread on methanol minimal agar containing 500 µg/ml of ampicillin. Resistant colonies were shown by alkaline denaturation lysates and gel electrophoresis to contain only pGSS15 plasmid. To demonstrate the potential of pGSS15, the murine dihydrofolate reductase (DHFR) gene, cloned in pBR322 from a homopolymeric tailed cDNA fragment by Chang et al (Nature *275* 617—624, 1978) has been transferred to AS1. The plasmid pDHFR7 obtained from S. Cohen, Stamford, was pBR322 containing the DHFR cDNA fragment cloned into the PstI site within the β-lactamase gene. The terminal transferase procedure used to clone the cDNA fragment regenerated PstI sites at either end, allowing the fragment to be cut out from pBR322 with PstI and separated by agarose gel electrophoresis. After staining the gel with ethidium bromide the band of DNA corresponding to the tailed cDNA fragment could be visualised under UV (330 nm) cut from the gel and the DNA recovered by electroelution in dialysis tubing. (The gel slice was placed inside dialysis tubing containing a small volume of 1/10 × tris acetate buffer. The tubing was just submerged in the same buffer in a tank with electrodes at either end. The DNA migrated out of the gel slice, into the buffer and up against the wall of the dialysis tubing after about 30—60 minutes at 100v. Reversal of the current for about 20 seconds freed the DNA off the tube and back into the buffer. The DNA solution was filtered through siliconised glass wool to remove any agarose, then passed through a 200 µl column of Sephadex DE-52 in tris acetate. The DNA became bound to the column matrix and was then eluted off in 2 × 100 µl and 1 × 50 µl of elution buffer, (2M NaCl, 50 mM Tris HCl, 1 mM EDTA).

Addition of 750 µl of absolute EtOH and chilling to −70°C for 5 minutes precipitated the purified fragment of DNA.

0.5 µg of pGSS15 DNA was linearised by digestion with PstI, mixed with 1 µg of the DHFR fragment and ligated *E.coli* WJB654 was transformed with the ligated mixture and plated on glucose minimal plates containing 10 µg/ml of trimethoprim. 216 out of 221 of the trimethoprim resistant clones were ampicillin sensitive, showing that the DHFR fragment had been inserted into that gene. Gel electrophoretic analysis of small scale cleared lysates showed that all the TpR clones tested on the same size, and larger than pGSS15, but that the ApsTps clones were also the same size. PstI digestion of DNA from both species revealed identical patterns, and EcoRI/BglII digests gave two different patterns corresponding to the DHFR fragment being in either orientation. However, only one orientation was functional, showing that transcription was from the β-lactamase promoter.

One such trimethoprim resistant clone designated pDHFR # 2.43 was transferred to AS1 in a manner exactly analogous to that described for pGSS15.

Mobilisation using R64drd 11 was preferred since this produced an AS1 strain carrying only the pDHFR plasmid.

DHFR assay

Cells of WJB654 (pDHFR # 2.43) were grown at 37°C overnight in glucose minimal medium containing 50 µg/ml methionine with or without 10 µg/ml trimethoprim. Cells of AS1 (pDHFR # 2.43) were grown at 37°C overnight in methanol minimal medium with or without 100 µg/ml trimethoprim. After harvesting the cells by centrifugation at 6K revs for 10 minutes, the pellets were resuspended in 3 vols 50 mM potassium phosphate buffer pH 7 then subjected to disruption by sonication (4—6 µ, 4 × 30 sec. bursts with 60 sec. intervals) on ice in the presence of 10 µM PMSF and 10 µM benzamidine. Debris was removed by centrifugation, 15 min at 10K revs, then 60 min at 35K revs.

The standard enzyme assay mixture contained: 50 µM dihydrofolate; 60 µM NADPH; 12 mM 2-mercaptoethanol; 50 mM potassium phosphate buffer pH 7.0, and enzyme (normally 100 µl of sonicate) in a final volume of 1 ml. Decrease in absorption at 340 nm was measured over a period of up to 10 min. Protein concentration was determined for each sample so that specific activities could be compared. The enzyme unit is described as that amount which causes a decrease in absorbancy of 0.010 per minute under the assay conditions described above.

TABLE 1
DHFR activities (units/mg protein)

| E.coli WJB654 | E.coli WJB654 (pDHFR) | AS1 | AS1 (pHHFR) |
|---|---|---|---|
| 0.92 | 1.6 | 2.79 | 25.00 |
| 0.48 | 0.53 | 2.80 | 16.60 |
| | | 2.17 | 10.40 |
| | | (i) 0 ) | (i) 2.4 ) |
| | | ) | ) |
| | | (ii) 0 ) | (ii) 0.48) |

The figures in brackets refer to the effect of (i) adding 100 µg/ml of trimethoprim to the enzyme assay. The AS1 activity is completely inhibited, as is expected for a bacterial enzyme, the AS1 (pDHFR # 2.43) activity is reduced to about 25%, in keeping with observations that the mammalian enzyme is less prone to inhibition by trimethoprim, and (ii) changing the substrate to folate instead of dihydrofolate, and again as expected the bacterial enzyme from AS1 was unable to use folate, but the AS1 containing the cloned mammalian gene could use it, although less efficiently than dihydrofolate.

pDHFR # 2.43 has also been transferred, via plate crosses into *Alcaligenes eutrophus* and *Methylomonas methylovora* where the cells are again rendered resistant to trimethoprim, and the plasmid has been shown to be present by alkaline denaturation isolation of DNA and electrophoresis.

Materials and Methods

*Bacterial strains*: WJB654 is *E.coli* met B, rk⁻, mk⁺, su³ obtained from W J Brammer, NM36 is *E.coli* pol A, rk⁺, mk⁺, su° obtained from C. Hadfield. The strains used for preparing *in vitro* BW162 is *E.coli* C600 Col IR *thr leu thi* Sms

Plasmids

| | | | |
|---|---|---|---|
| pBR322 | | 4.362 Kb | Ap Tc |
| R300B | Inc Q | 8.5 Kb non conjugative mob$^+$ | Su Sm |
| R483 | Inc Iα | tra$^+$ | Tp Sm |
| R388 · | Inc W | tra$^+$ | Tp Su |
| S-a | Inc W | tra$^+$ | CmKmSmSu |

Media

*E.coli* strains were grown in Luria Broth (LB) on Luria broth agar (LBA), or on glucose minimal agar (GMA).

LB: Difco Bacto Tryptone, 10 g; Difo Yeast Extract, 5 g; NaCl, 5 g; glucose, 1 g per litre adjusted to pH 7.2.

LBA: Difco Bacto Tryptone, 10 g; Difo Yeast Extract, 5 g; NaCl, 5 g; Difco agar, 15 g per litre adjusted to pH 7.2.

GMA: 1 × Spizizen salts*; 0.2% glucose; Difco agar, 15 per litre.

*Spizizen salts; $(NH_4)_2 SO_4$, 2 g; $K_2HPO_4$, 14 g; $KH_2PO_4$, 6 g; trisodium citrate, 1 g; $MgSO_4$. $7H_2O$, 0.2 g per litre.

AS1 was grown in methanol minimal broth (MMB) or on methanol minimal agar (MMA).

MMB: $(NH_4)_2SO_4$, 1.8 g; $MgSO_4.7H_2O$. 0.2 g; $NaH_2PO_4$, 5.6 g; $K_2HPO_4$, 7.6 g; 0.97% $FeCl_3$ solution, 0.1 ml; trace metal solution**, 1.0 ml per litre.

**Trade metal solution: $CuSO_4$. $5H_2O$, 0.02 g; $MnSO_4.4H_2O$, 0.01 g; $ZnSO_4.7H_2O$, 0.01 g; $CaCO_3$, 1.8 g; 1M HCl 36.6 ml per litre.

MMA: MMB + Difco agar, 15 g per litre.

Where necessary, amino acids were added to GMA at 50 mg per litre and antiobiotics were added where indicated at the followoing levels;

| Antibiotic | Symbol | Level used in µg/ml | |
|---|---|---|---|
| | | E.coli | ASI |
| Ampicillin | Ap | 30 | 100 |
| Chloramphenicol | Cm | 25*** | 25 |
| Kanamycin | Km | 10 | 25 |
| Streptomycin | Sm | 15 | 15 |
| Sulfonamide | Su | 1000 | |
| Tetracycline | Tc | 10 | 2—10 |
| Trimethoprim | Tp | 10*** | 100 |

*** not used in nutrient media.

Isolation of plasmid DNA

Cells from an 800 ml stationary phase culture of the plasmid containing strain were harvested by centrifugation then resuspended in 8 ml of ice cold 25% sucrose in 50 mM tris (hydroxymethyl)aminamethane (Tris), pH 8.0. Lysozyme was added (1.5 ml of a 10 mg/ml solution in water) and after 15 minutes on ice 1.5 ml of 0.5M ethylenediaminetetra-acetic acid (EDTA) pH 8.5 was added. After a further 15 min on ice lysis was brought about by the addition of 12 ml of 0.1% triton X-100 in 50 mM Tris pH 8.0, 62.5 mM EDTA pH 8.5. The lysate was then cleared by centrifugation at 18,000 rpm for 30 min and 20 ml of supernatant mixed with 19.5% cesium chloride and 1.25 ml of a 10 mg/ml solution of ethidium bromide. This mixture was centrifuged to equilibrium (40 hrs at 40,000 rpm in a Sorvall T865 rotor) when the lower plasmid DNA band could be visualised by UV light and, after removal of the chromosomal DNA, could be collected with a syringe and 18 gauge needle, by side puncture of the tube.

The ethidium bromide was removed by extraction with isopropanol that had been equilibrated with cesium chloride. After 2 hrs dialysis against TE buffer (10 mM Tris, 1 mM EDTA pH 7.2) the DNA solution

7

was extracted twice with equal volumes of phenol saturated with 0.5M Tris pH 8.0, then three times with ether. Excess ether was blown off, the solution made 0.3M in sodium acetate and the DNA was precipitated by addition of 0.54 volume of isopropanol.

Transformation of E.coli K-12 strains with plasmid DNA

*E.coli* cells were rendered competent by treatment with calcium as follows: a fresh overnight culture of the recipient strain was diluted 1:50 in LB and grown at 37°C with vigorous aeration, for approximately 100 min, then cooled rapidly on ice. The cells were pelleted by centrifugation at 4,000 rpm for 10 min and resuspended in 0.5 volume of ice cold 10 mM calcium chloride. After pelleting once more the cells were resuspended in 0.05 volume of 75 mM calcium chloride and kept on ice.

200µl of these competent cells were mixed with 0.1—1 µg of plasmid DNA in 100 µl of SSc (150 mM sodium chloride, 15 mM tri-sodium citrate) and incubated on ice for 30 min. The mixture was heated at 42°C for 2 min, followed by a further 20 min on ice. The cells were then diluted 1:10 in LB and incubated at 37°C for 45—60 min, to allow expression of plasmid encoded functions, then were concentrated by centrifugation and resuspended in 100 µl of bacterial buffer ($KH_2PO_4$, 3 g; $Na_2HPO_4$, 7 g; NaCl, 4 g; $MgSO_4.7H_2O$ 0.2 g per litre) before being plated out on selective media. This procedure typically produced $10^4$—$10^6$ antibiotic resistant colonies per µg of plasmid DNA.

Partial digests using restriction endonucleases

10 µg of plasmid DNA were incubated with 5 units of restriction enzyme (where 1 unit is defined as that amount of enzyme that will completely digest 1 µg of DNA at 37°C in one hour) at 37°C in a total volume of 200 µl containing the buffer recommended by the enzyme supplier. At times, 0, 5, 10, 20, 30, 45, 60 and 90 min from the addition of the enzyme, 20 µl samples of the reaction mixture were remomved and heated to 70°C for 10 min to stop the reaction. 5 µl aliquots of each sample were then analysed by agarose gel electrophoresis.

Comparison of the partially digested DNA to totally digested or linearised plasmid DNA will give an indication as to the extent of digestion in each sample.

Ligations

Typically, total DNA concentration was adjusted to 5—30 µg/ml by diluting 2—15 µg of DNA in TN buffer (0.01M tris HCl pH 7.5; 0.1M NaCl). For a two-species ligation the DNA was incubated at 30°C for 15 min, for self-annealing this was omitted. The buffer was made to 66 mM tris HCl pH 7.4; 10 mM $MgCl_2$; 10 mM β-mercaptoethanol; 1 mM ATP and 100 µg/ml gelatin, by addition of one tenth of a volume of a 10X concentrated, freshly prepared, stock, and then T4 DNA ligase added. (The ligase was a gift of C. R. Wilson and B. Ely and was used at 1—3 µl/µg DNA). The mixture was incubated at 10°C for 16—48 hour then used to transform *E.coli*.

Mini cleared lysate isolation of plasmid DNA

A 10 ml culture of the plasmid containing strain was grown overnight in media (LB or minimal) containing an antibiotic as required. The cells were harvestd by centrifugation at 6K revs for 10 min then resuspended in 150 µl of ice-cold 25% sucrose in 50 mM tris HCl pH 8.0. 50 µl of lysozyme solution (10 mg/ml in water) were added and mixed gently on ice for 5 min. 50 µl of 0.5 EDTA pH 8.5 were added and mixed for a further 5 min on ice. Lysis of the cells was brought about after 15 min by the addition of 250 µl of triton solution (1 ml 0.1% Triton X-100 in 50 mM Tris pH 8.0, 62.5 mM EDTA pH 8.5). The lystate was cleared by centrifugation in an Eppendorph centrifuge for 15 min and the supernatant extracted three times with equal volumes of phenol that had been equilibrated with 0.5M Tris HCl pH 8.0. One further extraction with n-butanol, followed by two extractions with diethyl ether, was performed before making the solution of 0.3M in sodium acetate and precipitating the DNA with 0.54 volume of isopropanol and chilling to −70°C for 5 min. The DNA was collected by centrifuging in an Eppendorph, decanting the supernatant and freeze-drying the pellet. Addition of 40 µl of water produced a DNA solution that could be used for transformation, restriction and electrophoresis.

Preparation of Plasmid DNA by the Alkaline Extraction Method

(Birnboim & Doly, Nuc.Acid.Res. 7, 1513—1523, 1979) 10 ml cultures were grown and harvested as described above. The cells were resuspended in 1 ml of lysis solution (lysozyme 2 mg/ml, 25 mM Tris HCl pH 8.0, 10 mM EDTA and 50 mM glucose) and kept at 0°C for 30 min. 2 ml of alkaline SDS solution (1% SDS in 0.2N NaOH) were added and kept at 0°C for a further 5 min, when 1.5 ml of high salt solution (3M sodium acetate pH 4.8) were added. After a further 60 min at 0°C the mixture was centrifuged at 15K rev for 10 min and 4 ml of supernatant were carefully removed. Addition of 10 ml of cold absolute ethanol and chilling to −70°C for 5 min precipitated the DNA which was collected by centrifugation at 15K rev for 15 min. The pellet was redissolved in 1 ml of acetate/tris (0.1M sodium acetate, 50 mM Tris HCl pH 8.0) and 2 ml cold absolute ethanol added. After chilling and centrifuging as above, the pellet was redissolved in 200 µl water to give a DNA solution suitable for transformations, restrictions and electrophoresis. This method was preferred for rapidly preparing plasmid DNA from organisms other than *E.coli*.

Electrophoresis

0.5—1% agarose gels were used as 4 mm thick, 12.5 × 25 cm horizontal slabs. Electrophoresis buffer contained 40 mM Tris, 20 mM sodium acetate, 2 mM EDTA adjusted to pH 7.8 with acetic acid. Prior to loading, one tenth of a volume of 20% ficol containing the dye bromophenol blue, was added. Electrophoresis was normally at a constant voltage of 150v for 2—3 hour or 40v overnight. After electrophoresis, gels were stained with ethidium bromide (1 µg/ml) and photographed under UV illumination.

Protein Assay (Micro Lowry)

Reagents: 1% sodium potassium tartrate, 2% sodium carbonate, 0.5% copper sulfate, Folin reagent.

Method:

Mix sodium carbonate, sodium potassium tartrate and copper sulfate solutions in the raltio 49:1:1. Take 10 µl aliquote of sample and add water to make 100 µl. Add 100 µl 1M NaOH and leave for 10 min. Add 1 ml of copper sulfate reagent (prepared above). Leave for 10 min. Add 100 µl Folin reagent, diluted 1:1 with water, to vortexed solution. Leave for 30 min, then read $OD_{660}$ against water. The readings were compared to a standard curve made using Bovine Serum Albumin solutions containing 5, 10, 20, 30, 40, 50, 60, 75 and 100 µg of protein.

BRL Restriction enzyme assay conditions

ENDO R. Ava 1

30 mM NaCl
20 mM Tris-HCl (pH 7.4)
10 mM $MgCl_2$
DNA
Enzyme
Assay 37°C

EDNO. R. Bam HI

20 mM Tris-HCl (pH 8.0)
7 mM MgCl
100 mM NaCl
2 mM 2-mercaptoethanol
assay 37°C
DNA
enzyme

ENDO R. Bgl II

20 mM Tris HCl (pH 7.4)
7 mM $MgCl_2$
7 mM 2-mercaptoethanol
assay 37°C

ENDO R. Eco RI

100 mM Tris HCl (pH 7.2)
5 mM $MgCl_2$
2 mM 2-mercaptoethanol
50 mM NaCl
assay 37°C
DNA
enzyme

ENDO R—Hae II

50 mM Tris-HCl (pH 7.5)
5 mM $MgCl_2$
0.5 mM dithiothreitol
assay 37°C
DNA
enzyme

ENDO R. Hinc II (Hind II)

10 mM Tris HCl (pH 7.9)
1 mM dithiothreitol
60 mM NaCl
6.6 mM $MgCl_2$
assay 37°C
1.0 µg of DNA
Enzyme

ENDO R. Hind III

20 mM Tris HCl (pH 7.4)
7 mM $MgCl_2$
60 mM NaCl
assay 37°C
DNA
enzyme

ENDO R. Pst I

20 mM Tris-HCl (pH 7.5)
10 mM $MgCl_2$
50 mM $(NH_4)_2SO_4$
100 µg/ml BSA
DNA
Enzyme
Assay 37°C

9

| ENDO R. Sal I | ENDO R. Sst I |
|---|---|
| 8 mM Tris HCl (pH 7.6) | 14 mM Tris HCl (pH 7.5) |
| 6 mM MgCl$_2$ | 6 mM MgCl$_2$ |
| 0.2 mM Na$_2$ EDTA | 6 mM 2-mercaptoethanol |
| 150 mM NaCl | 90 mM NaCl |
| assay 37°C | assay 37°C |
| DNA | DNA |
| enzyme | enzyme |

New England Biolabs
restriction enzymes

*Pvu* I
    Assay conditions: 150 mM NaCl, 6 mM Tris-HCl (pH 7.4), 6 mM MgCl$_2$, 6 mM 2-mercaptoethanol, 100 μg/ml bovine serum albumin and DNA.
*Pvu* II
    Assay conditions: 60 mM NaCl, 6 mM Tris-HCl (pH 7.5), 6 mM MgCl$_2$, 6 mM 2-mercaptoethanol, 100 μg/ml bovine serum albumin and DNA.
    The preceding description is exemplative of the methods that may be employed in the production of composite plasmids. It illustrates the derivation of a series of vectros from R300B using pBR322 as a source of additional genes. pBR322 is particularly useful as it has been sequenced and it is well understood.
    The process is illustrated schematically in all following diagram and in detail in subsequent Figures 1, 2 and 3.

Figure 1

Construction of pGSS6

The plasmid R300B is shown arbitrarily linearised at the EcoRI site and pBR322 is shown linearised by HaeII. The dotted lines show the site of insertion to form the co-integrate pGSS6.

Figure ii

Formation of pGSS8 and pGSS9 following EcoRI digestion of pGSS6.

Fig. iii

Construction of pGSS15 by insertion of the HaeII Ap fragment from pBR322 into partially HaeII digested pGSS8. The HaeII site, within the Tc gene of pGSS8, is shown to highlight the fortuitous reconstruction of the gene. pGSS15 has lost its SstI and AvaI sites close to Sm, has become Sm$^s$ and has gained an extra SalI site.

Thus according to another aspect of the invention there is provided a composite plasmid or cloning vector obtained by cleaving the plasmid R300B and effecting ligation in the presence of another cleaved plasmid comprising factors which it is desired to introduce into R300B, to generate a cointegrate (composite plasmid).

According to a further aspect of the invention there is provided a method of introducing into a bacterium, particularly· an autotrophic or methylotrophic bacterium, genetic material foreign to that bacterium and capable of effecting expression in the form of a metabolite, particularly a metabolite having biological activity in a different organism, by infecting the cell with a composite plasmid carrying the said foreign genetic material.

A genetically modified organism according to the invention may be employed in a variety of applications, depending, of course, upon its physiology and upon the nature of the inserted DNA.

Most conveniently it will be used in a biosynthetic mode, in a suitable nutrient medium, metabolite being passed out of the cell into the surrounding culture solution and extracted therefrom in the conventional way. Alternatively it may be cultured and harvested as the intact cell with subsequent extraction of the cells after separation from the medium. Such techniques are well known and require no further explanation.

Examples of metabolites that may usefully be expressed by the organisms of the invention as a consequence of the introduction of a foreign, eukaryotic gene include insulin and human growth hormone as well as other hormones and antibiotic materials, e.g. penecillius. Many other potentially valuable metabolites will be apparent to the skilled worker in this field.

Thus, in a further embodiment of the invention there is provided a method of effecting biological synthesis using a methylotrophic or an autotrohic bacterium comprising inserted foreign, particularly eukaryotic genetic material.

In particular there is provided a method of effecting biological synthesis using M. methylophilus comprising inserted foreign, particularly eukaryotic genetic material.

The following example illustrates the insertion into AS1 of genetic material coding for ovalbumin, and expression of·this protein.

## Example

### Outline

The protein ovalbumin, which can be identified by immuno-logical methods, was used to demonstrate translation of inserted DNA at the Pst endonuclease site of plasmid pGS15. The double stranded CDNA of the ovalbumin structural gene was isolated from the plasmid pov230 by restriction endonuclease *Taq* 1 cutting and extraction of the ovalbumin structural gene DNA from an agarose gel. The isolated ovalbumin CDNA was oligo(dG)-oligo(dC) tailed into the Pst endonuclease site of pGS15, which lies within the β-lactamase gene. The hybrid DNA was used to transform *E.coli* PA340.22. Transformants, selected using L.agar containing tetracycline (10 μg/ml) which were found to be sensitive to Ampicillin (25 μg/ml), were screened for the presence of ovalbumin DNA, using a modified colony hybridization method. Positive clones from the hybridization were screened for the production of ovalbumin using an immunoradiometric assay. The plasmid R751 was used to mobilise hybrid plasmids from ovalbumin producing *E.coli* strains into *Methylophilus methylotrophus (AS1)*. Tetracycline resistant clones (1 μg/ml in methanol minimum agar) were screened for the production of ovalbumin.

### Construction of Hybrid DNA Molecules

The plasmid pov230 was constructed by the insertion of the ovalbumin structural gene (cDNA) into the plasmid pMB9. The restriction endonuclease *Taq* 1 was used to cut the plasmid pov230 and the ovalbumin structural gene DNA (Taq-OV) was extracted from an agarose gel using hydroxyl apatite. pGS15 DNA was linearised with the restriction endonuclease *Pst* 1.

The following tubes were prepared:

15

| Tube | DNA | 10 × Restriction endonuclease buffer | H₂O | Enzyme |
|------|-----|-----|-----|--------|
| 1 | pov230 (7.5 µg in 30 µl) | 4 µl | 2 µl | 4 µl *Taq* 1 (∾8 units) |
| 2 | pMB9 (2 µg in 30 µl) | 4 µl | 5 µl | 1 µl *Taq* 1 (∾2 units) |
| 3 | pGS15 (5 µg in 21 µl) | 4 µl | 11 µl | 4 µl *Pst* 1 (∾5 units) |

Tubes 1 and 2 were incubated at 65°C for 1 hour and Tube 3 at 37°C for 1 hour. Approximately 0.2 µg of restricted DNA was run on an agarose gel alongside 0.2 µg of untreated DNA for each of the plasmids to check restriction completion.

Isolation of Taq-ovalbumin structural gene (cDNA)

1. The *Taq*i cut pOV230 DNA was run on a 1.5% agarose gel containing 1 µg/ml ethidium bromide for approximatley two hours.

2. The gel was viewed using ultra violet light and a narrow trough was cut into the agar immediately behind the required DNA bond. (The DNA band common to both pMB9 and pOV230 (slowest running band) was cut to leave the trough).

3. The trough was filled with hydroxyl apatite, preswollen in Tris acetate buffer. The current was reversed so that the required DNA band was run into the hydroxyl apatite (∾15 mins, 150 V).

4. The hydroxyl apatite was transferred to a column of Sephadex G50 fine, prepared in a siliconised glass pasteur pipette and equilibrated with Trisacetate buffer.

5. The DNA was eluted with IM sodium phosphate buffer pH 7.5 and 200 µl fractions collected. The fractions containing the DNA were detected by drying a 10 µl aliquot from each onto an ethidium bromide (1 µg/ml) agarose (2%) plate and viewing under UV light. The DNA, eluted in fraction 5, with a volume of 200 µl was phenol and ether extracted. 0.11 volume of 3M sodium acetate pH 5 and 0.54 volume of isopropyl alcohol was added to the extracted DNA and left overnight at −20°C.

6. The DNA was centrifuged at 12,000 rpm for 5 minutes and the pellet washed with excess 80% ethanol. After recentrifuging the ethanol was removed under vacuum and the DNA resuspended in 200 µl of Tris-EDTA buffer.

7. An aliquot (5 µl) of the isolated DNA was run on an agarose gel alongside the *Taq*i cut pOV230 parent plasmid, in order to confirm purity of the isolated fragment.

Terminal addition of homopolymer tracts to double-stranded DNA

A method described by Roychandhury et al was used to add homopolymer tracts of deoxyribonucleotide Guanidine to the 3′ ends of linearised pGS15 DNA. Similarly homopolymer tracts of deoxyribonucleotide Cytosine were added to the isolated 'Taq-OV' DNA.

Two reaction tubes were prepared;

1. [3H]dGTP tailing of Pst 1 cut pGS12 DNA 5 µCi of [3H]dGTP was dried under vacuum (5 µl) in a 1.5 ml tube. (Specific activity 9 Ci/mM). The following were added to the tube:

4 µg of *Pst*1 cut pGS15 DNA (32 µl).

144 µl H₂O.

2 µl CoCl₂ (100 mM).

2 µl Bovine Serum albumin (5 mg/ml).

20 µl Terminal transferase reaction buffer.

The total volume in the tube was 200 µl. Reactants were mixed and preincubated at 37°C for 30 minutes prior to the addition of terminal transferase enzyme. (1 µl ∾8 units)

2. [3H] dCTP tailing of isolated ovalbumin structural gene cDNA

10 µCi of [3H] dCTP was dried under vacuum (10 µl) in a 1.5 ml tube (specific activity 18.4 Ci/mM). The following were added to the tube;

0.5 µg isolated DNA (30 µl).

141 µl H₂O.

2 µl CoC12 (100 mM).

2 µl Bovine serum albumin (5 mg/ml).

20 µl Terminal transferase reaction buffer.

The total volume in the tube was 200 µl. Reactants were mixed and preincubated at 37°C for 30 minutes prior to the addition of terminal transferase enzyme (1 µl ∾8 units). At times intervals 1 µl samples were removed from the two reaction tubes and each transferred to a tube containing 0.4 ml of salmon sperm

16

DNA (0.5 mg/ml in 0.2M NaPP pH 7.0). 150 µl of 50% cold Trichloroacetic acid (TCA) was added to each tube and the tubes were kept on ice for at least 10 minutes. Each sample was washed through a 2.1 cm diam. glass Microfibre filter (GFC) (Whatman) with 50 ml of 5% TCA, followed by 15 ml of ethanol. Filters were dried in glass counting vials at 65°C for 10 minutes at 10 ml of toluene/PPO was added to each vial. Samples were counted. The counts incorporated into the DNA were followed with each sample taken. The reaction in each tube was allowed to continue until a tail of 20 bases was added to the DNA samples.

Considering the molecular size of the DNA used and the relative amount of $^3H$ labelled deoxyribonucleoside triphosphate it was calculated for pGS15 DNA that a value of 2010 dpm 1 µl sample corresponded to the addition of 20 bases (i.e. 100.5 dpm/µl/base).

Similarly for the isolated 'Taq-OV' DNA it was calculated that a value of 6180 dpm/µl sample corresponded to the addition of 20 bases (i.e. 309 dpm/µl/base).

When these respective values were obtained the reactions were stopped by the addition of 10 µl 0.5M EDTA.

Isolating tailed DNA

The dG-tailed pGS15 DNA and dC-tailed 'Taq-OV' DNA were isolated from other reactants by gel filtration on Sephadex.

A sephadex G50 fine column was prepared using a siliconised glass pasteur pipette. Tailed DNA was eluted with Tris-EDTA buffer and 200 µl fractions collected. 5 µl samples of each fraction were pipetted into 2 ml of scintillation fluid and counted. Fractions containing the first peak of activity, indicating tailed-DNA were pooled.

Annealing dC-tailed and dG-tailed DNA

The dG-tailed pGS15 DNA and dC-tailed 'Taq-OV' DNA were mixed in equimolar amounts to a final DNA concentration of 1 µg/ml in Tris-EDTA buffer. The mixture was made 1M with NaCl. The tube containing the annealing mixture was placed in a water bath at 65°C for 15 minutes. The water bath was turned off and allowed to cool slowly overnight.

Transformation

The hybrid DNA obtained was used to transform *E.coli* PA.340.22.

Preparation of Component cells

1. 1 ml of PA340.22 culture grown overnight at 37°C with shaking was pipetted into 100 mls of L.broth. Cells were incubated, with shaking, at 37°C until an O.D. (E550 nm) of 0.5 was reached. Cells were cooled on ice for 30 minutes.

2. Cells were harvested at 4°C and 2,000 rpm for 30 minutes, washed in 0.5 volume of 0.1 M $CaCl_2$ and resuspended in 5 mls of 0.1 M cold $CaCl_2$.

The following transformation mixture was prepared in 1.5 ml Eppendorf tubes:

10 µl DNA.

5 µl of 2OX standard saline citrate.

200 µl competent cells.

85 µl double distilled water.

Tubes were left on ice for 1 hour, placed in a water bath at 42°C for 2 minutes, then returned to ice for 1 hour. Each transmission mixture was transferred to 5 mls of L. broth and incubated, with shaking at 37°C for 30 minutes.

Cells were plated onto L.agar containing tetracycline (10 µg/ml). The Pst endonuclease site of pGS15 lies within the β-lactamase gene which confers resistance to Ampicillin. Transformants found resistant to tetracycline were tested for sensitivity to Ampiccillin (25 µg/ml in L.agar).

Nick Translation labelling of DNA

20 µCi of ($\alpha^{32}P$)-dCTP was dried under vaccum into a 1.5 ml Ependorph tube. The following solutions were then added:

2 µl 0.1 mM dTTP.

2 µl 0.1 mM dGTP.

2 µl 0.1 mM dATP.

25 µl TAQOV DNA (1 µg).

5 µl 10 × Nick translation buffer (0.5M Tris; 50 mM.$MgCl_2$; 100 mM βMSH; pH 8).

14 µl $H_2O$.

The reaction was started by the addition of 1 unit of DNA polymerase I (Kornberg polymerase) (from Boehringer) ($\sim$ 0.5 µl), followed by gentle mixing, centrifugation to remove bubbles and incubation at 15°C for 90 minutes. The reaction was stopped by the addition of 100 µl Nick translation stop buffer (10 mM Tris; 10 mM EDTA; ½% SDS; 0.2 mg/ml yeast t-RNA, pH 8), followed by phenol and ether extractios.

The DNA was separated from the unreacted triphosphates on a Sephadex G50 fine column as above (Section "isolating tailed DNA").

Colony Hybridisation

Nitrocellulose filters (Schleicher & Schnell, 82 mm, BA85) were autoclaved for 15 minutes between filter papers wrapped in tin foil. The filters were then dried at 65°C for 2 hours.

A sterile filter was carefully laid onto the sugar surface of a petri-dish, containing tetracycline (10 µg/ml). Cultures or colonies to be screened for a particular DNA sequence were tooth-picked onto the surface of the filter, in a noted pattern. The petri-dish and filter were incubated overnight at 37°C and colonies grew on the surface of the filter.

After growth the filter was carefully removed from the agar, and placed colony side up, in turn onto filter papers wet with:

1. 0.5M. NaOH for 7 min.
2. 1M. Tris pH 7.4 for 2 min.
3. 1M. Tris pH 7.4 for 2 min.
4. 3M. NaCl, 1M. Tris pH 7.4 for 4 min.

The filter was then dried for 2 hours at 80°C under vacuum.

Prior to hybridisation the filter was washed for 3 hours at 65°C in Denhardts solution. The filter was then placed in a 9 cm glass petri-dish and 30 ml of degassed Denhardts solution added at 65°C. Approximately $10^6$ Cherenkov cpm of the nick translated DNA probe, in 0.5 ml degassed Denhardts solutioon was boiled for 10 minutes, and added to the petri-dish containing the filter while hot. The dish was then sealed with tape, and incubated overnight at 65°C.

After incubation the filter was washed 4 times in Denhardt's solution containing 1% SDS for 15 minutes each at 65°C, and finally for 1 hour in 3 × SSC + 1% SDS at 65°C. The filter was then dried at 65°C for 3 hours.

The filter was autoradiographed overnight using Kodac X-omatic H films and a Kodac intensifying screen. After developing, colonies which contained the required sequence were shown as dark spots on the X-ray film.

Immunoradiometric assay

Antibody molecules adsorb strongly to the plastic polystyrene and are not significantly dislodged by washing. This was the basis for an immunoradiometric assay used to determine the production of ovalbumin by *E.coli* and AS1 cells harbouring hybrid plasmid DNA.

Preparation of cell free extracts for E.coli transformants

A single colony was transferred to 5 ml of L.broth containing 10 µg/ml of tetracycline and grown overnight, with agitation at 37°C. A 100 µl aliquot was serially diluted and plated to give viable counts per ml of culture. Cells were harvested at 2000 rpm for 10 minutes and washed in 5 ml of phosphate buffered saline (PBS). Cells were resuspended in 5 ml of cold PBS and sonicated 6 × 15 seconds on ice using an MSE sonicator at maximum amplitude (Model no. 150W). Cell debris was centrifuged at 2,000 rpm for 10 minutes and the cell free supernatant was used for the assay. Sonicated cell free extract of PA340.22 was used as a control.

Polystyrene wells (7 mm diameter by 12 mm deep) were coated with anti ovalbumin immunoglobulin. 300 µl of 10 µg/ml antibody in 0.2M $NaHCO_3$ pH 9.2 was pipetted into each well and incubated at 37°C for 5 hours. Wells were washed three times for 5 minutes each with wash buffer (PBS with 0.5% normal rabbit serum and 0.1% Bovine serum albumin). In triplicate 300 µl of sonicated cell extract was pipetted into wells and released antigen allowed to bind overnight at 4°C. A standard antigen (sigma ovalbumin) was used, diluted in PBS.

Following incubation, wells were washed three times and 300 µl of $^{125}$I anti-ovalbumin, immuno-globulin was added (25 ng/ml $^{125}$I anti-ovalbumin, specific activity 18 µCi/µg) diluted in wash buffer. Wells were incubated at 37°C for 3 hours and washed. Individual wells were counted on a Gamma counter (LKB minigamma).

A standard curve for the ovalbumin samples was constructed and the cell extracts compared with this. As a measure of the immunoreactive ovalbumin-like material produced in *E.coli* and *AS1* strains containing hydbrid plasmids, the dilution of each strain giving 50% of the maximum binding was taken to be equivalent to the mass of authentic material with the same counts. The viable cell count per ml of overnight culture and the equivalent amount of ovalbumin detected were used to calculate the number of molecules of ovalbumin-like protein produced per cell.

| Strain | Molecules of ovalbumin produced/cell |
|---|---|
| PA340.22 (PGS15-Taqov) | 28 molecules per cell |
| PA340.22 (pOMP2) | 22 molecules per cell |
| AS1 (PGS15-Taqov) | 44 molecules per cell |

**Claims**

1. An autotrophic or methylotrophic bacterium which
(a) comprises foreign genetic material which (i) in its unmodified state the bacterium would not comprise and (ii) has been inserted into a naturally occurring or composite plasmid and
(b) is capable of effecting expression of the said foreign genetic material with generation of a polypeptide which the foreign genetic material encodes and which in its unmodified state the bacterium would not generate with the proviso that the foreign genetic material is not a microbial g*d*h gene.

2. Autotrophic or methylotrophic bacterium according to claim 1 which is a species of Methylophilus, Methylomonas, Alcaligenes, or Pseudomonas.

3. A bacterium according to claim 2 in which the said species is Methylophilus methylotrophus, Methylomonas methylovora, Methylomonas clara, Methylomonas methanolica, Alicaligenes eutrophus, Pseudomonas methanolica, Pseudomonas methylonica, Pseudomonas utilis or Pseudomonas inaudita.

4. A bacterium according to claim 2 in which the said species is Methylophilus methylotrophus, Methylomonas methylovora or Alicaligenes eutrophus.

5. A bacterium according to claim 2 in which the said species is Methylophilus methylotrophus.

6. A bacterium according to any one of the preceding claims in which the foreign DNA is eukaryotic DNA.

7. A bacterium according to claim 6 in which the DNA is mammalian DNA.

8. A bacterium according to claim 7 in which the DNA is human DNA.

9. A bacterium according to any one of the preceding claims in which the foreign DNA encodes a hormone, or an antiviral material.

10. A method for the introduction of foreign genetic material into a cell of an autotrophic or methylotrophic bacterium characterised in that the cell is infected with a plasmid or derived from a plasmid of one of the incompatibility groups N, P, Q or W into which the foreign genetic material has been inserted, with the proviso that the foreign genetic material is not a microbial g*d*h gene.

11. A method according to claim 10 in which the plasmid is a composite plasmid.

12. A method according to claim 11 in which the composite plasmid is derived from R300B.

13. A biosynthetic method comprising the step of culturing a bacterium according to any one of claims 1 to 9 under conditions such that expression of the foreign genetic material occurs.


**Patentansprüche**

1. Autotrophes oder methylotrophes Bakterium, das
(a) genetisches Fremdmaterial aufweist, welches (i) das Bakterium in seinem unmodifizierten Zustand nicht umfassen würde und (ii) in ein natürlich vorkommendes oder zusammengesetztes Plasmid eingefügt wurde, und
(b) zum Ausdruck des genetischen Fremdmaterials bei der Erzeugung eines Polypeptids befähigt ist, das durch das genetische Fremdmaterial kondiert wird und durch das Bakterium in seinem unmodifizierten Zustand nicht erzeugt werden würde unter der Bedingung, daß das genetische Fremdmaterial kein mikrobisches g*d*h-Gen ist.

2. Autotrophes oder methylotrophes Bakterium nach Anspruch 1, das eine Spezies des Methylophilus, Methylomonas, Alcaligenes oder Pseudomonas ist.

3. Bakterium nach Anspruch 2, bei dem die Spezies Methylophilus methylotrophus, Methylomonas methylovora, Methylomonas clara, Methylomonas methanolica, Alcaligenes eutrophus, Pseudomonas methanolica, Pseudomonas methylonica, Pseudomonas utilis oder Pseudomonas inaudita ist.

4. Bakterium nach Anspruch 2, bei dem die Spezies Methylophilus methylotrophus, Methylomonas methylovora oder Alicaligenes eutrophus ist.

5. Bakterium nach Anspruch 2, bei dem die Spezies Methylophilus methylotrophus ist.

6. Bakterium nach einem der vorhergehenden Ansprüche, bei dem die Fremd-DNS eukaryotische DNS ist.

7. Bakterium nach Anpsruch 6, bei dem die DNS Säugetier-DNS ist.

8. Bakterium nach Anpsruch 7, bei dem die DNS menschliche DNS ist.

9. Bakterium nach einem der vorhergehenden Ansprüche, bei dem die Fremd-DNS ein Hormon oder ein antivirales Material kodiert.

10. Verfahren zur Einführung von genetischem Fremdmaterial in eine Zelle eines autotrophen oder methylotrophen Bakteriums, dadurch gekennzeichnet, daß die Zelle mit einem Plasmid einer der Unverträglichketisgruppen N, P, Q oder W infiziert oder von einem solchen Plasmid abgeleitet wird, in das genetisches Fremdmaterial eingefügt wurder unter der Voraussetzung, daß das genetische Fremdmaterial kein mikrobisches g*d*h-Gen ist.

11. Verfahren nach Anpsruch 10, bei dem das Plasmid ein zusammengesetztes Plasmid ist.

12. Verfahren nach Anspruch 11, bei dem das zusammengesetzte Plasmid von R300B abgeleitet ist.

13. Biosynthetisches Verfahren, bei dem man ein Bakterium nach irgendeinem der Ansprüche 1 bis 9 unter solchen Bedingungen züchtet, daß Ausdruck des genetisches Fremdmaterials erfolgt.

# 0 037 273

**Revendications**

1. Bactérie autotrophe ou méthylotrophe qui

(a) comprend un matériel génétique étranger que (i) dans son état non modifié, la bactérie ne comporterait pas et qui (ii) a été inséré dans un plasmide naturel ou composite, et

(b) est capable d'effecteur l'expression dudit matériel génétique étranger en engendrant un polypeptide pour lequel code le matériel génétique étranger et que, dans son état non modifié, la bactérie n'engendrerait pas, sous réserve que la matériel génétique étranger ne soit pas un gène microbien g*d*h.

2. Bactérie autotrophe ou méthylotrophe suivant la revendication 1, qui est une espèce appartenant au genre Methylophilus, Methylomonas, Alcaligenes ou Pseudomonas.

3. Bactérie suivant la revendication 2, dont l'espèce est Methylophilus methylotrophus, Methylomonas methylovora, Methylomonas clara, Methylomonas methanolica, Alcaligenes eutrophus, Pseudomonas methanolica, Pseudomonas methylonica, Pseudomonas utilis ou Pseudomonas inaudita.

4. Bactérie suivant la revendication 2, dont ladite espèce est Methylophilus methylotrophus, Methylomonas methylovora ou Alcaligenes eutrophus.

5. Bactérie suivant la revendication 2, dont ladite espèce est Methylophilus methylotrophus.

6. Bactérie suivant l'une quelconque des revendications précédentes, dans laquelle l'ADN étranger est un ADN d'eukaryote.

7. Bactérie suivant la revendication 6, dans laquelle l'ADN est un ADN de mammifère.

8. Bactérie suivant la revendication 7, dans laquelle l'ADN est de l'ADN humain.

9. Bactérie suivant l'une quelconque des revendications précédentes, dans laquelle l'ADN étranger code pour une hormone ou pour une matière antivirale.

10. Procédé pour introduire un matériel génétique étranger dans une cellule d'une bactérie autotrophe ou méthylotrophe, caractérisé en ce que la cellule est infectée avec un plasmide ou est dérivée d'un plasmide de l'un les groupes d'incompatibilité N, P, Q ou W dans lesquels le matériel génétique étranger a été inséré, sous réserve que la matériel génétique étranger ne soint pas un gène microbien g*d*h.

11. Procédé suivant la revendication 10, dans lequel le plasmide est un plasmide composite.

12. Procédé suivant la revendication 11, dans lequel le plasmide composite est dérivé de R300B.

13. Procédé biosynthétique, qui comprend l'étape consistant à cultiver une bactérie suivant l'une quelconque des revendications 1 à 9 dans des conditions telles que l'expression du matériel génétique étranger ait lieu.

20